Europäisches Patentamt

European Patent Office

Office européen des brevets

(11) Veröffentlichungsnummer: **0 445 330 A1**

## (12) EUROPÄISCHE PATENTANMELDUNG

(21) Anmeldenummer: **90104373.7**

(22) Anmeldetag: **07.03.90**

(51) Int. Cl.⁵: **A61B 6/10, F16P 3/12**

(43) Veröffentlichungstag der Anmeldung:
**11.09.91 Patentblatt 91/37**

(84) Benannte Vertragsstaaten:
**BE DE FR GB IT**

(71) Anmelder: **Siemens Aktiengesellschaft**
**Wittelsbacherplatz 2**
**W-8000 München 2(DE)**

(72) Erfinder: **Goldhorn, Klaus, Dipl.-Ing. (FH)**
**Ganghoferweg 3**
**W-8520 Erlangen(DE)**

(54) **Medizinisches Gerät mit einem in Richtung wenigstens eines Freiheitsgrades relativ zu einer Fläche motorisch verstellbaren Geräteteil.**

(57) Medizinisches Gerät mit einem in Richtung wenigstens eines Freiheitsgrades (x, alpha) relativ zu einer Fläche (3) motorisch verstellbaren Geräteteil (2) und mit einer eine Anzahl von im Verstellbereich des Geräteteiles (2) an der Fläche (3) anbringbaren Schaltplatten (7, 8) aufweisenden Schaltplatteneinrichtung (9), deren Schaltplatten (7, 8) bei der Anwesenheit eines Objektes im Bereich der jeweiligen Schaltplatte (7, 8) jeweils ein Signal abgeben, wobei die von den Schaltplatten (7, 8) abgegebenen Signale in Abhängigkeit von einer gewünschten Verstellrichtung in der Weise ausgewertet werden, daß nur die in der gewünschten Verstellrichtung liegende(n) Schaltplatte(n) (7, 8) berücksichtigt wird (werden), und ein Verstellen des Geräteteiles (2) in der gewünschten Verstellrichtung unterbunden wird, wenn wenigstens eine berücksichtigte Schaltplatte (7, 8) ein die Anwesenheit eines Objektes anzeigendes Signal abgibt.

FIG 1

EP 0 445 330 A1

Die Erfindung betrifft ein medizinisches Gerät mit einem in Richtung wenigstens eines Freiheitsgrades relativ zu einer Fläche motorisch verstellbaren Geräteteil, mit einer Steuereinrichtung zum Verstellen des Geräteteiles und mit einer an der Fläche anbringbaren Schaltplatteneinrichtung, die bei Anwesenheit eines Objektes im Bereich der Schaltplatteneinrichtung ein Signal abgibt, welches der Steuereinrichtung zugeführt ist, die bei Anwesenheit des Signals ein Verstellen des Geräteteiles unterbindet. Dabei soll der Begriff "Objekt" sowohl Lebewesen als auch Gegenstände umfassen.

Bei derartigen Geräten, es kann sich hierbei um Diagnose- oder Therapiegeräte handeln, besteht stets Verletzungsgefahr für das Bedienpersonal und eventuell auch die Patienten, da deren Körper, insbesondere deren Extremitäten, zwischen dem verstellbaren Geräteteil und der Fläche eingeklemmt werden können. Außerdem besteht die Gefahr von Beschädigungen des Gerätes, wenn sich ein Fremdkörper zwischen dem verstellbaren Geräteteil und der Fläche befindet. Man ist deshalb bestrebt, die Verletzungsgefahr für das Bedienpersonal und für die Patienten sowie die Gefahr von Beschädigungen des Gerätes auszuschalten. Als Lösungen gegen diese Gefahren sind z.B. an der jeweiligen Fläche anzubringende Markierungen sowie Schaltplatten (DE-AS 21 49 240, DE-PS 21 48 760 und DE-OS 38 11 380) bekannt. Markierungen aber sind nur optische Hinweise, die nicht immer beachtet werden. Schaltplatten können in ihrer Größe und Anordnung auf der Fläche nur einen Kompromiß darstellen. Zum einen besteht die Möglichkeit, den gesamten gefährdeten Bereich abzudecken, mit der Folge, daß beispielsweise infolge einer die Schaltplatteneinrichtung betretenden Person störende Unterbrechungen bzw. Blockierungen der Gerätebewegungen auftreten, ohne daß tatsächlich eine Gefahr für die Person gegeben ist. Wenn dieser Nachteil vermieden werden soll, besteht zum anderen die Möglichkeit, die Schaltplatte zu verkleinern, mit der Folge, daß nun nicht mehr der gesamte gefährdete Bereich in ausreichendem Maße gesichert ist.

Der Erfindung liegt die Aufgabe zugrunde, ein Gerät der eingangs genannten Art so auszubilden, daß eine Sicherung des gesamten gefährdeten Bereiches gewährleistet ist und dennoch Unterbrechungen bzw. Blockierungen der Bewegung des Geräteteiles ohne Vorliegen einer unmittelbaren Gefahr sicher vermieden sind.

Nach der Erfindung wird diese Aufgabe gelöst durch ein medizinisches Gerät mit einem in Richtung wenigstens eines Freiheitsgrades relativ zu einer Fläche motorisch verstellbaren Geräteteil, mit einer Steuereinrichtung zum Verstellen des Geräteteiles und mit einer eine Anzahl von im Verstellbereich des Geräteteiles an der Fläche anbringbaren Schaltplatten aufweisenden Schaltplatteneinrichtung, deren Schaltplatten bei Anwesenheit eines Objektes im Bereich der jeweiligen Schaltplatte jeweils ein Signal abgeben, wobei die Signale der einzelnen Schaltplatten der Steuereinrichtung zugeführt sind und wobei die Steuereinrichtung in Abhängigkeit von einer gewünschten Verstellrichtung die von den Schaltplatten abgegebenen Signale in der Weise auswertet, daß sie nur die in der gewünschten Verstellrichtung liegende(n) Schaltplatte(n) berücksichtigt, und ein Verstellen des Geräteteiles in der gewünschten Verstellrichtung unterbindet, wenn wenigstens eine berücksichtigte Schaltplatte ein die Anwesenheit eines Objektes anzeigendes Signal abgibt. Im Falle der Erfindung wird also die Bewegung des Geräteteiles nicht bereits dann unterbunden, d.h. unterbrochen oder blockiert, wenn sich ein Objekt an beliebiger Stelle in dem gesamten gefährdeten Bereich befindet, sondern erst dann, wenn es eine solche Position einnimmt, daß die Bewegung des Geräteteiles in Richtung auf das Objekt erfolgt. Dies wird dadurch erreicht, daß die Steuereinrichtung nicht ständig die Ausgangssignale sämtlicher Schaltplatten berücksichtigt, sondern nur die Ausgangssignale derjenigen Schaltplatten, die für eine gewünschte Verstellrichtung tatsächlich relevant sind. Im Gegensatz zum Stand der Technik stellt also die erfindungsgemäße Lösung keinen Kompromiß der genannten Art dar, sondern ermöglicht eine vollständige Sicherung des gefährdeten Bereiches, ohne daß die Gefahr von unnötigen Störungen der Bewegung des Geräteteiles besteht.

Eine besonders bevorzugte Variante der Erfindung sieht vor, daß Mittel zum Bestimmen der momentanen Stellung des Geräteteiles vorgesehen sind, deren der Stellung des Geräteteiles entsprechende Ausgangssignale der Steuereinrichtung zugeführt sind, und daß die Steuereinrichtung die von den Schaltplatten abgegebenen Signale in der Weise auswertet, daß sie anhand der Ausgangssignale der Mittel zum Bestimmen der momentanen Stellung des Geräteteiles diejenige(n) Schaltplatte(n) berücksichtigt, die dem Geräteteil in Richtung der gewünschten Verstellrichtung unmittelbar benachbart ist (sind). Durch diese Maßnahme ist die Gefahr von an sich unnötigen Störungen der Bewegung des Geräteteiles nochmals weiter vermindert, da die Verstellung des Geräteteiles nicht bereits dann unterbunden wird, wenn sich, wenn auch in einem größeren Abstand von dem Geräteteil, in der gewünschten Verstellrichtung ein Objekt auf der Schaltplatteneinrichtung befindet, sondern nur dann, wenn sich ein Objekt in Verstellrichtung in unmittelbarer Nähe des Geräteteiles befindet. Die hierfür erforderlichen Mittel zum Bestimmen der momentanen Stellung des Geräteteiles können durch herkömmliche Weggeber gebildet sein. Falls das Geräteteil durch Schrittmotore verstellt wird, können die Mittel zum Bestimmen der momentanen Stellung des Geräteteiles auch durch die Ansteuerschaltung für die Schrittmotore gebildet sein, deren Ausgangssignale dann die momentane Stellung des Geräteteiles kennzeichnen.

Die Schaltplatten der Schalteinrichtung bilden vorzugsweise ein matrixartiges Raster, wobei dann mit zunehmend feiner Unterteilung des Rasters die Gefahr von unnötigen Beeinträchtigungen der Bewegung des Geräteteiles abnimmt, während gleichzeitig der für die Schaltplatteneinrichtung erforderliche Aufwand steigt, da eine größere Anzahl von Schaltplatten erforderlich ist.

Ein einfacher, funktionssicherer und sich ändernden Bedürfnissen leicht anpaßbarer Aufbau der Steuereinrichtung ist gewährleistet, wenn nach einer Variante der Erfindung vorgesehen ist, daß die Steuereinrichtung einen Speicher umfaßt, in dem für eine Vielzahl von Stellungen des Geräteteiles und für die ausgehend von diesen Stellungen jeweils mögliche(n) Verstellrichtung(en) des Geräteteiles die zu berücksichtigende(n) Schaltplatte(n) gespeichert ist (sind). Im Falle einer derartigen Steuereinrichtung wird bzw. werden also in Abhängigkeit von den Ausgangssignalen der Mittel zum Bestimmen der momentanen Stellung des Geräteteiles dann, wenn eine Verstellung des Geräteteiles erfolgen soll, unter Berücksichtigung der gewünschten Verstellrichtung die zu berücksichtigende Schaltplatte bzw. die zu berücksichtigenden Schaltplatten aus dem Speicher abgerufen.

Die Schaltplatteneinrichtung kann druckempfindliche und/oder kapazitiv wirkende Schaltplatten enthalten, wobei letztere den Vorteil bieten, daß sie nicht nur dann, wenn sich ein Objekt auf der Schaltplatte befindet, ein Signal liefern, sondern bereits dann, wenn sich ein Objekt im Bereich der Schaltplatte befindet, ohne diese zu berühren. Als druckempfindliche Schaltplatten kommen z.B. elektromechanische Schaltplatten in Frage, wie sie in der DE-PS 21 48 760 beschrieben sind. Als kapazitiv wirkende Schaltplatten können die in der DE-OS 23 11 380 beschriebenen Schaltplatten Verwendung finden.

In der Regel wird vorgesehen sein, daß die Schaltplatteneinrichtung am Boden oder an einer Wand des Aufstellungsraumes des medizinischen Gerätes anbringbar ist. Es sind aber auch solche Fälle denkbar, in denen eine Anbringung der Schaltplatteneinrichtung an einer Fläche des Gerätes selbst erfolgt, relativ zu welcher das Geräteteil verstellbar ist.

Ausführungsbeispiele der Erfindung sind in den beigefügten Zeichnungen beschrieben. Es zeigen:

Fig. 1    in stark schematisierter Darstellung ein erfindungsgemäßes medizinisches Gerät,

Fig. 2 bis 4    unterschiedliche Betriebszustände einer weiteren Ausführungsform eines erfindungsgemäßen Gerätes, und

Fig. 5    ein Blockschaltbild des Gerätes nach den Fig. 2 bis 4.

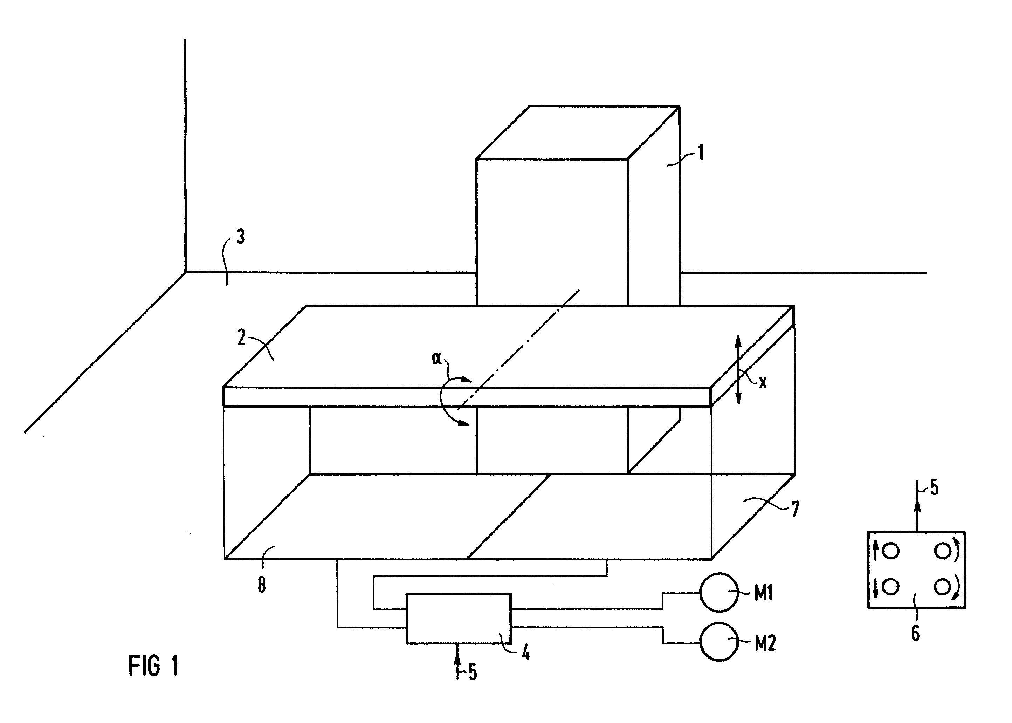

In der Fig. 1 ist ein medizinisches Gerät, beispielsweise ein urologischer Arbeitsplatz, gezeigt, von dem nur eine Tragsäule 1 und ein an dieser angebrachter Behandlungstisch 2 dargestellt sind. Der Behandlungstisch 2 ist an der fest am Boden 3 des Behandlungsraumes verankerten Tragsäule 1 derart angebracht, daß er in Richtung des geradlinigen Doppelpfeiles y höhenverstellbar und um die horizontal und quer zur Längsachse des Behandlungstisches 2 verlaufende Achse A in Richtung des gekrümmten Doppelpfeiles "alpha" schwenkbar ist. Die Verstellung des Behandlungstisches 2 in den genannten Richtungen erfolgt motorisch mittels zweier schematisch angedeuteter Elektromotore M1, M2, die in an sich bekannter, nicht dargestellter Weise über geeignete Getriebe auf den Behandlungstisch 2 einwirken.

Um den Behandlungstisch 2 in der gewünschten Weise verstellen zu können, ist eine an eine Steuereinrichtung 4 mittels der Leitung 5 angeschlossene Tastatur 6 vorgesehen. An der linken Seite der Tastatur 6 ist ein erstes Tastenpaar vorgesehen, dessen obere Taste dazu dient, mittels des Motors M1 eine Aufwärtsbewegung des Behandlungstisches 2 in der Richtung y zu bewirken, so wie dies durch einen neben der Taste angebrachten Pfeil angedeutet ist. Die untere Taste des ersten Tastenpaares dient in entsprechender Weise dazu, eine Absenkung des Behandlungstisches 2 in der Richtung y zu bewirken. Ein zweites Tastenpaar ist auf der rechten Seite der Tastatur 6 vorgesehen. Die obere Taste des zweiten Tastenpaares dient dazu, mittels des Motors M2 eine Schwenkbewegung des Behandlungstisches 2 in Richtung des Doppelpfeiles "alpha" gegen den Uhrzeigersinn zu bewirken. Die untere Taste des zweiten Tastenpaares ist in entsprechender Weise vorgesehen, um eine Schwenkbewegung des Behandlungstisches im Uhrzeigersinn bewirken zu können. Auch dem zweiten Tastenpaar sind die jeweilige Tastenfunktion anzeigende Pfeile zugeordnet.

Unterhalb des Behandlungstisches 2 sind auf dem Boden 3 des Behandlungsraumes zwei Schaltplatten 7, 8 angebracht, bei denen es sich um druckempfindliche elektromechanische Schaltplatten der in der DE-PS 21 48 760 beschriebenen Art handelt. Die Schaltplatten 7, 8 bedecken diejenige Fläche des Bodens 3, die der Parallelprojektion des waagerecht stehenden Behandlungstisches 2 mit rechtwinklig zu dem Boden 3 verlaufenden Strahlen, diese sind für die Eckpunkte des Behandlungstisches 2 in dünnen Linien angedeutet, entspricht. Die Projektion der Achse A entspricht der Trennlinie zwischen den Schaltplatten 7 und 8. Die druckempfindlichen Schaltplatten 7, 8 geben jeweils ein Signal ab, wenn sich auf ihnen ein Objekt befindet, dessen Gewicht ausreichend hoch ist, um in den Schaltplatten 7, 8 befindliche elektrische Kontakte zu schließen. Die Schaltplatten 7 und 8 sind über entsprechende Leitungen mit der Steuereinrichtung 4 verbunden.

Die Steuereinrichtung 4 ist derart ausgebildet, daß sie auf die Betätigung einer der Tasten der Tastatur 6 hin die Motore M1 und M2 nur dann in der erforderlichen Weise ansteuert, wenn entweder durch Verstellen des Behandlungstisches 2 in der gewünschten Richtung das Einklemmen eines Objektes zwischen dem Behandlungstisch 2 und dem Boden 3 des Behandlungsraumes grundsätzlich ausgeschlossen ist, oder anhand der von den Schaltplatten 7 und/oder 8 gelieferten Signale feststeht, daß sich zwischen dem Behandlungstisch 2 und dem Boden 3 kein Objekt befindet, das bei Bewegung des Behandlungstisches 2 in der gewünschten Richtung eingeklemmt werden könnte. Sofern die Steuereinrichtung 4 eine bestimmte Bewegung zuläßt, erfolgt eine entsprechende Ansteuerung des entsprechenden Motors M1 bzw. M2, solange die entsprechende Taste der Tastatur 6 gedrückt ist, es sei denn, nicht dargestellte, an sich bekannte Endlagenschalter sprechen an, bevor die Betätigung der Tastatur beendet wird.

Dies bedeutet, daß die Steuereinrichtung 4, wenn eine Aufwärtsbewegung des Behandlungstisches 2 in der Richtung y gewünscht wird, den Motor M1 nach Betätigung der entsprechenden Taste der Tastatur 6 ohne weiteres in der erforderlichen Richtung ansteuert. Bei den verbleibenden Bewegungen des Behandlungstisches 2 überprüft die Steuereinrichtung 4, bevor sie den Motor M1 bzw. M2 in der erforderlichen Weise ansteuert, zunächst anhand der von den Schaltplatten 7 und 8 gelieferten Signale, ob zwischen dem Behandlungstisch 2 und dem Boden 3 ein Objekt vorhanden ist, das bei Ausführung der gewünschten Bewegung zwischen Behandlungstisch 2 und Boden 3 eingeklemmt werden könnte. Während eine gewünschte Bewegung ausgeführt wird, überwacht die Steuereinrichtung 4 weiterhin die Signale der Schaltplatten 7 und 8, um die weitere Ausführung der gewünschten Bewegung unterbrechen zu können, falls während der Ausführung dieser Bewegung die Anwesenheit eines Objektes angezeigt wird, das durch die gewünschte Bewegung zwischen dem Behandlungstisch 2 und dem Boden 3 eingeklemmt werden könnte. Im einzelnen berücksichtigt die Steuereinrichtung 4 beim Absenken des Behandlungstisches 2 in der Richtung y die Signale der beiden Schaltplatten 7 und 8, d.h. nur wenn keine der beiden Schaltplatten 7 und 8 die Anwesenheit eines Objektes anzeigt, ist eine Ansteuerung des Motors M1 im Sinne einer Absenkung des Behandlungstisches 2 möglich. Soll ein Schwenken des Behandlungstisches 2 im Uhrzeigersinn erfolgen, berücksichtigt die Steuereinrichtung 4 nur das Ausgangssignal der Schaltplatte 7, da ein im Bereich der Schaltplatte 8 befindliches Objekt beim Schwenken des Behandlungstisches 2 im Uhrzeigersinn nicht eingeklemmt werden kann. Nur wenn kein die Anwesenheit eines Objektes anzeigendes Signal der Schaltplatte 7 auftritt, ist eine Ansteuerung des Motors M2 in der erforderlichen Richtung möglich. Das Vorstehende gilt sinngemäß für ein Schwenken des Behandlungstisches 2 gegen den Uhrzeigersinn, mit dem einzigen Unterschied, daß die Steuereinrichtung 4 nun das Signal der Schaltplatte 8 berücksichtigt.

Es wird also deutlich, daß im Gegensatz zum Stand der Technik im Falle der Erfindung bei Anwesenheit eines Objektes auf der Schaltplatteneinrichtung eine gewünschte Bewegung des Behandlungstisches 2 nur dann unterbunden wird, wenn tatsächlich ein Einklemmen des Objektes zwischen Behandlungstisch 2 und Boden 3 möglich ist.

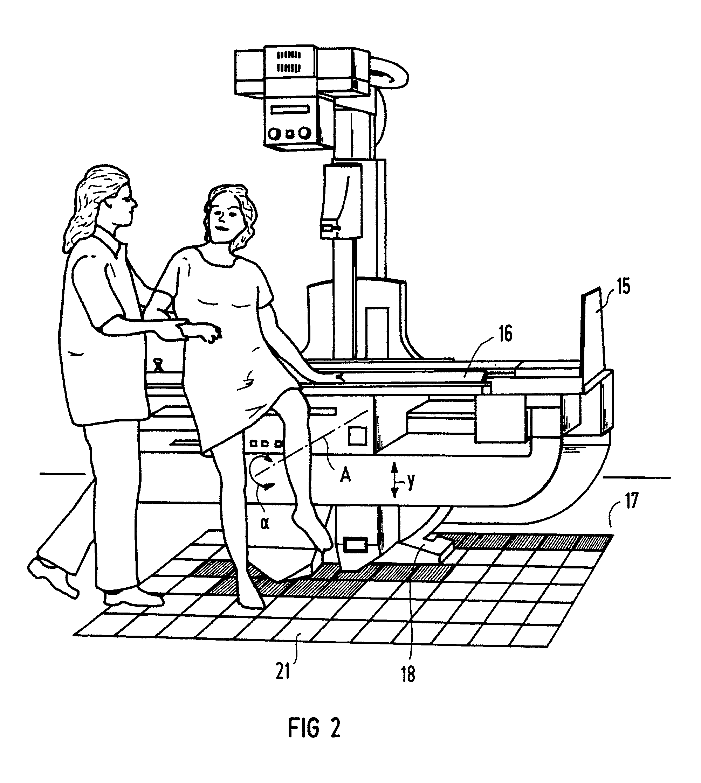

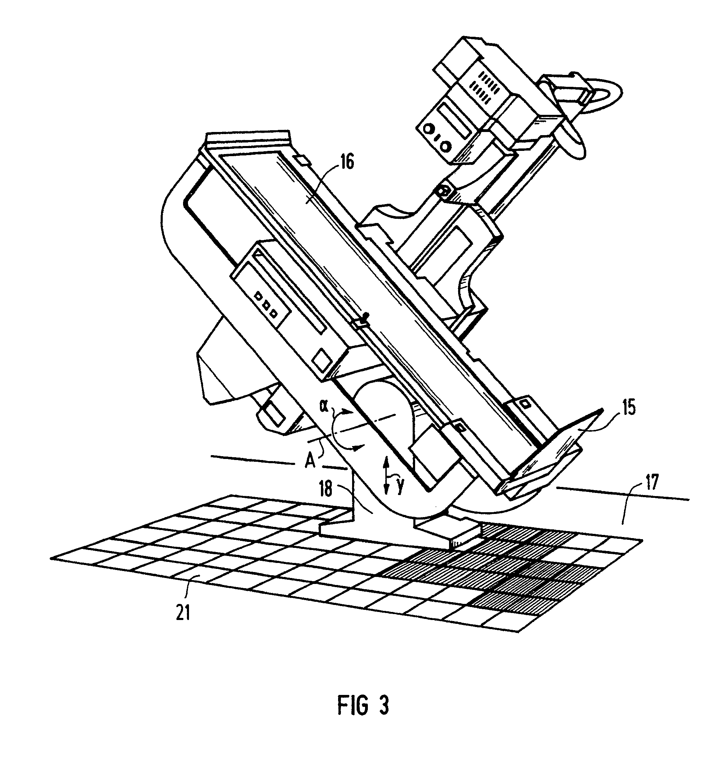

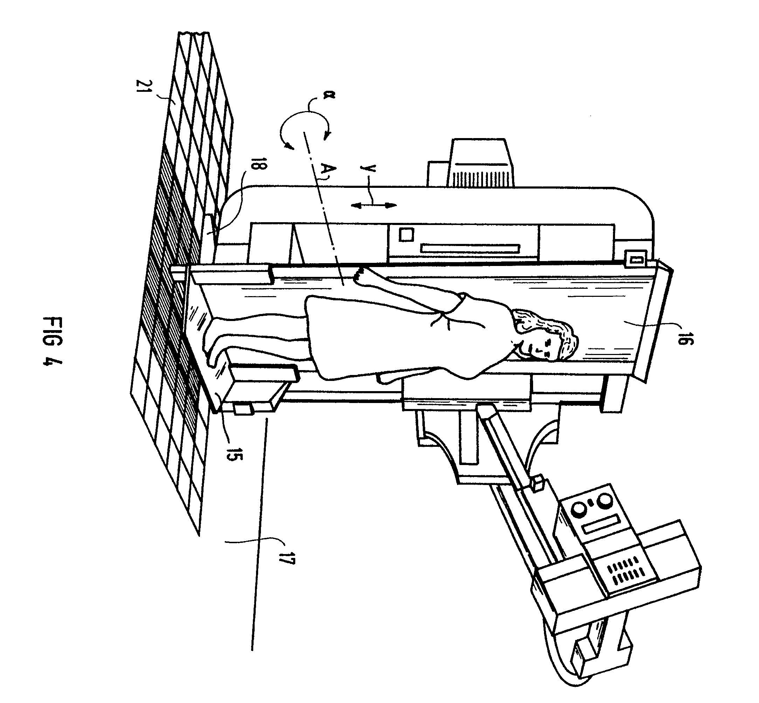

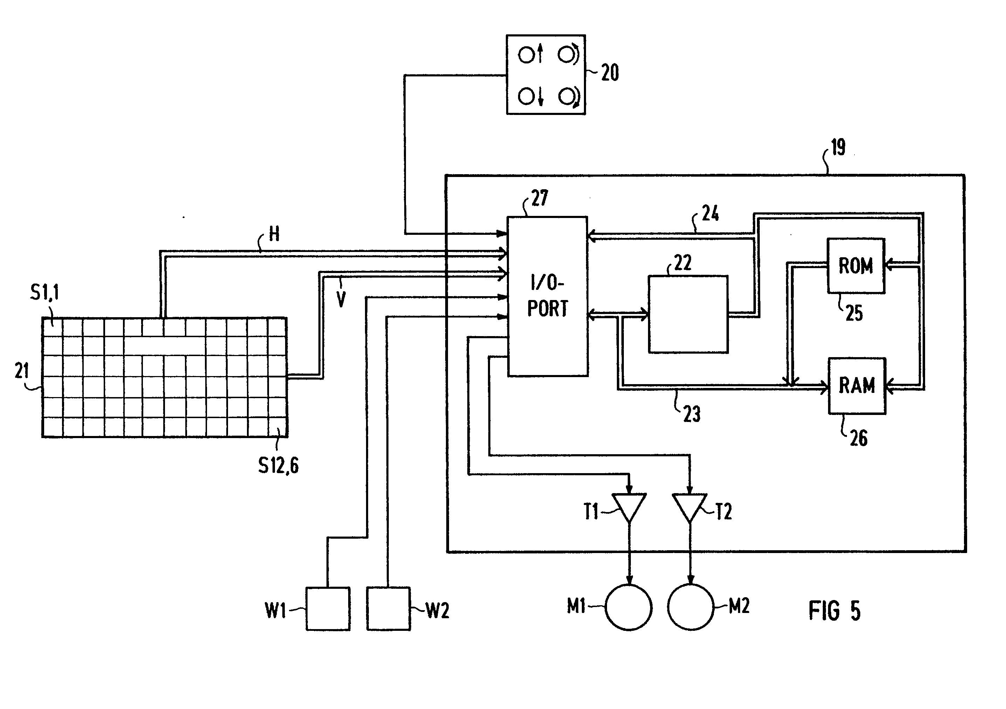

Ein weiteres Ausführungsbeispiel ist anhand der Fig. 2 bis 5 verdeutlicht. Diese Fig. zeigen ein an sich bekanntes Röntgenuntersuchungsgerät, dessen Funktion hier nur insoweit von Interesse ist, als dessen mit einer Fußbank 15 versehener Patientenlagerungstisch 16 motorisch verstellbar an einem auf dem Boden 17 des Untersuchungsraumes befestigten Sockel 18 angebracht ist. Im einzelnen ist der Patientenlagerungstisch 16 mittels eines Elektromotors M1 in nicht dargestellter, an sich bekannter Weise in Richtung des geradlinigen Doppelpfeils y höhenverstellbar an dem Sockel 18 angebracht. In ebenfalls nicht dargestellter und an sich bekannter Weise ist der Patientenlagerungstisch 16 mit Hilfe des Elektromotors M2 in Richtung des gekrümmten Doppelpfeiles "alpha" um eine quer zur Längsachse des Patientenlagerungstisches 16 und horizontal verlaufende Achse A schwenkbar.

Die Steuerung der Bewegung des Patientenlagerungstisches 16 erfolgt mittels einer Steuereinrichtung 19, die die Elektromotore M1 und M2 in der Weise ansteuert, wie dies die Betätigung einer an die Steuereinrichtung 19 angeschlossenen Tastatur 20 erforderlich macht. Die Tastatur 20 weist für jede Verstellrichtung des Patientenlagerungstisches 16 ein durch entsprechende Pfeilsymbole gekennzeichnetes Tastenpaar auf, dessen Tasten dazu dienen, die jeweilige Bewegung in der einen oder der anderen Richtung zu bewirken. Außer der Tastatur 20 und den Elektromotoren M1 und M2 sind zwei Weggeber W1, W2 an die Steuereinrichtung 19 angeschlossen, die der Stellung des Patientenlagerungstisches 16 relativ zu dem Sockel 18 und damit relativ zu dem Boden 17 entsprechende elektrische Signale liefern, wobei der Weggeber W1 für die Richtung y und der Weggeber W2 für die Richtung "alpha" zuständig ist.

Weiter ist an die Steuereinrichtung 19 eine Schaltplatteneinrichtung 21 angeschlossen, die wie aus den Fig. 2 bis 4 ersichtlich ist, im Bereich des Röntgenuntersuchungsgerätes am Boden 17 des Untersuchungsraumes angebracht ist. Die Schaltplatteneinrichtung 21 weist eine Vielzahl von in Matrixform rasterartig in sechs Zeilen und zwölf Spalten angeordnete Schaltplatten S1,1 bis S12,6 auf. Dabei bezeichnet die erste Ziffer die Spalte und die zweite Ziffer die Zeile der Schaltplatteneinrichtung 21, in der sich eine Schaltplatte befindet. Die Positionen S5,2 bis S8,2 sind nicht besetzt, da sich hier der Sockel 18 befindet. Die Schaltplatten S1,1 bis S12,6, bei denen es sich um kapazitiv wirkende Schaltplatten handelt, die gemäß der DE-OS 38 11 380 ausgebildet sein können, geben jeweils dann ein Signal ab, wenn sich ein Objekt in ihrem Bereich befindet. Die Schaltplatteneinrichtung 21 ist über zwei busartig zusammengefaßte Leitungsstränge H und V mit der Steuereinrichtung 19 derart verbunden, daß jede der Schaltplatten S1,1 bis S12,6, die ein die Anwesenheit eines Objektes anzeigendes Signal liefert, von der Steuereinrichtung 19 eindeutig identifiziert werden kann. Die Schaltplatteneinrichtung 21 dient dazu, Bewegungen des Patientenlagerungstisches zu unterbinden, die zu Verletzungen einer Person bzw. Beschädigungen des Röntgenuntersuchungsgerätes führen könnten.

Die Steuereinrichtung 19 enthält in an sich bekannter Weise einen Mikroprozessor 22, der über einen Datenbus 23 und einen Adreßbus 24 mit einem Nur-Lese-Speicher (ROM) 25, einem Schreib-Lese-Speicher (RAM) 26 und einer Eingangs/Ausgangs-Beschaltung (I/O-PORT) 27 verbunden ist. An den I/O-PORT 27 sind die Tastatur 20, die Schaltplatteneinrichtung 21, die Weggeber W1 und W2 und die Elektromotore M1 bis M2 angeschlossen, wobei den Elektromotoren M1 bis M2 entsprechende Treiberstufen T1 und T2 zugeordnet sind. Neben einem die im folgenden beschriebene Arbeitsweise der Steuereinrichtung 19 gewährleistenden Steuerprogramm ist (sind) in dem ROM 25 für eine Vielzahl von Stellungen des Patientenlagerungstisches 16 relativ zu dem Sockel 18 und für die ausgehend von diesen Stellungen jeweils möglichen Verstellrichtungen des Patientenlagerungstisches 16 die zu berücksichtigende(n) Schaltplatte(n) gespeichert. In dem RAM 26 werden beim Betrieb des Gerätes anfallende Daten zwischengespeichert.

Wird eine der Tasten der Tastatur 20 betätigt, erhält der Mikroprozessor 22 über den I/O-PORT 27 Daten, die es ihm ermöglichen, die betätigte Taste der Tastatur 20 zu identifizieren. Im Anschluß hieran nimmt der Mikroprozessor 22 über den I/O-PORT 27 den Ausgangssignalen der Weggeber W1 und W2 entsprechende Daten auf, anhand derer er die momentane Stellung des Patientenlagerungstisches 16 relativ zu dem Sockel 18 ermittelt. Unter Berücksichtigung dieser Stellung und der gewünschten Verstellrichtung, d.h. der betätigten Taste der Tastatur 20, ruft der Mikroprozessor 22 aus dem ROM 25 Daten ab, die angeben, welche der Schaltplatten S1,1 bis S12,6 der Schaltplatteneinrichtung 21 für die ausgehend von der momentanen Position des Patientenlagerungstisches 16 gewünschte Verstellrichtung des Patientenlagerungstisches 16 zu berücksichtigen sind, um eine Gefährdung, sei es die Gefährdung einer Person durch den sich bewegenden Patientenlagerungstisch 16 oder eine Gefährdung des Patientenlagerungstisches 16 selbst durch Anstoßen an einem Fremdkörper, ausschließen zu können. Über den I/O-PORT 27 fragt der Mikroprozessor 22, nachdem er die entsprechenden Daten aus dem ROM 25 gelesen hat, die zu berücksichtigenden Schaltplatten der Schaltplatteneinrichtung 21 ab. Sofern keine zu berücksichtigende Schaltplatte ein die Anwesenheit eines Objektes anzeigendes Signal liefert, steuert der Mikroprozessor 22 den für die gewünschte Bewegung des Patientenlagerungstisches 16 maßgeblichen Motor M1 bzw. M2 in der entsprechenden Richtung an. Hierdurch ändert sich die Position des Patientenlagerungstisches 16 relativ zu dem Sockel 18, was auch zu einer Änderung der Ausgangssignale der Weggeber W1, W2 führt. Die sich ändernden Ausgangssignale der Weggeber überwacht der Mikroprozessor 22 während der gesamten Zeitdauer, für die eine Taste der Tastatur 20 betätigt ist. Während dieser Zeitdauer ruft der Mikroprozessor 22 fortlaufend die der sich ändernden Position des Patientenlagerungstisches 16 und der gewählten Bewegungsrichtung entsprechenden zu berücksichtigende(n) Schaltplatte(n) aus dem ROM 25 ab und fragt in der zuvor beschriebenen Weise die jeweils zu berücksichtigende(n) Schaltplatte(n) ab. Es wird also deutlich, daß während der Bewegung des Patientenlagerungstisches 16 mit der sich ändernden Position des Patientenlagerungstisches 16 unter Umständen nach und nach andere Schaltplatten von der Steuereinrichtung 19 berücksichtigt werden als zu Beginn der Bewegung.

Liefert keine zu berücksichtigende Schaltplatte ein auf die Anwesenheit eines Objektes hindeutendes Signal, wird die gewünschte Bewegung fortgesetzt, solange die entsprechende Taste der Tastatur 20 gedrückt ist, es sei denn, zuvor sprechen nicht dargestellte, an sich bekannte Endlagenschalter an. Stellt der Mikroprozessor 22 im Anschluß an das Drücken einer Taste der Tastatur 20 fest, daß eine dem Patientenlagerungstisch 16 in der gewünschten Verstellrichtung unmittelbar benachbarte Schaltplatte ein auf die Anwesenheit eines Objektes hindeutendes Signal abgibt, wird die gewünschte Bewegung so lange nicht eingeleitet, bis das Objekt sich entfernt hat bzw. entfernt wurde. Liefert eine zu berücksichtigende Schaltplatte, während bereits eine Bewegung des Patientenlagerungstisches 16 ausgeführt wird, ein die Anwesenheit eines Objektes anzeigendes Signal, unterbricht der Mikroprozessor 22 die Bewegung, indem er die weitere Ansteuerung des entsprechenden Motors M1 bzw. M2 unterbindet.

Wie anhand der Fig. 2 bis 4, die ein Aufrichten des zunächst horizontalen Patientenlagerungstisches 16 in eine vertikale Position verdeutlichen, leicht vorstellbar ist, wandert also während der Bewegung ein sich veränderndes Muster von zu berücksichtigenden Schaltplatten, die im Falle der Fig. 2 bis 4 durch eine Schraffur hervorgehoben sind, über die Schaltplatteneinrichtung 21, wobei jeweils diejenigen Schaltplatten zu berücksichtigende Schaltplatten sind, die dem Patientenlagerungstisch 16 unmittelbar benachbart sind, wobei unter unmittelbar benachbart diejenigen Teile bzw. Bereiche des Patientenlagerungstisches verstanden werden sollen, deren Abstand von dem Boden 17 rechtwinklig zu dem Boden 17 gemessen einen Mindestabstand unterschreitet.

Es wird deutlich, daß im Falle des zuletzt beschriebenen Ausführungsbeispieles im Vergleich zu dem zuerst beschriebenen die Gefahr von an sich unnötigen Störungen der Bewegung des Patientenlagerungstisches nochmals weiter vermindert ist, da die Verstellung des Patientenlagerungstisches 16 nicht bereits dann unterbunden ist, wenn sich, wenn auch in einem größeren Abstand von dem Patientenlagerungstisch 16, in der jeweils gewünschten Verstellrichtung ein Objekt auf der Schaltplatteneinrichtung 21 befindet, sondern nur dann, wenn sich ein Objekt in der gewünschten Verstellrichtung in unmittelbarer Nähe des Patientenlagerungstisches 16 befindet.

**Patentansprüche**

1. Medizinisches Gerät mit einem in Richtung wenigstens eines Freiheitsgrades (y, alpha) relativ zu einer Fläche (3; 17) motorisch verstellbaren Geräteteil (2; 16), mit einer Steuereinrichtung (4; 19) zum Verstellen des Geräteteiles (2; 16), und mit einer eine Anzahl von im Verstellbereich des Geräteteiles (2; 16) an der Fläche (3; 17) anbringbaren Schaltplatten (7, 8; S1,1 bis S12,6) aufweisen den Schaltplatteneinrichtung (9; 21), deren Schaltplatten (7, 8; S1,1 bis S12,6) bei Anwesenheit eines Objektes im Bereich der jeweiligen Schaltplatte (7, 8; S1,1 bis S12,6) jeweils ein Signal abgeben, wobei die Signale der einzelnen Schaltplatten (7, 8; S1,1 bis S12,6) der Steuereinrichtung (4; 19) zugeführt sind und wobei die Steuereinrichtung (4; 19) in Abhängigkeit von einer gewünschten Verstellrichtung die von den Schaltplatten (7, 8; S1,1 bis S12,6) abgegebenen Signale in der Weise auswertet, daß sie nur die in der gewünschten Verstellrichtung liegende(n) Schaltplatte(n) (7, 8; S1,1 bis S12,6) berücksichtigt, und ein Verstellen des Geräteteiles (2; 16) in der gewünschten Verstellrichtung unterbindet, wenn wenigstens eine berücksichtigte Schaltplatte (7, 8; S1,1 bis S12,6) ein die Anwesenheit eines Objektes anzeigendes Signal abgibt.

2. Medizinisches Gerät nach Anspruch 1, **dadurch gekennzeichnet,** daß Mittel (W1, W2) zum Bestimmen der momentanen Stellung des Geräteteiles (16) vorgesehen sind, deren der Stellung des Geräteteiles (16) entsprechende Ausgangssignale der Steuereinrichtung (19) zugeführt sind, und daß die Steuereinrichtung (19) die von den Schaltplatten (S1,1 bis S12,6) abgegebenen Signale in der Weise auswertet, daß sie anhand der Ausgangssignale der Mittel (W1, W2) zum Bestimmen der momentanen Stellung des Geräteteiles (16) diejenige(n) Schaltplatte(n) berücksichtigt, die dem Geräteteil (16) in Richtung der gewünschten Verstellrichtung unmittelbar benachbart ist (sind).

3. Medizinisches Gerät nach Anspruch 1 oder 2, **dadurch gekennzeichnet,** daß die Schaltplatten (S1,1 bis S12,6) der Schaltplatteneinrichtung (21) ein matrixartiges Raster bilden.

4. Medizinisches Gerät nach einem der Ansprüche 1 bis 3, **dadurch gekennzeichnet,** daß die Steuereinrichtung (19) einen Speicher (25) umfaßt, in dem für eine Vielzahl von Stellungen des Geräteteiles (16) und für die ausgehend von diesen Stellungen jeweils mögliche(n) Verstellrichtung(en) des Geräteteiles (16) die zu berücksichtigende(n) Schaltplatte (n) (S1,1 bis S12,6) gespeichert ist (sind).

5. Medizinisches Gerät nach einem der Ansprüche 1 bis 4, **dadurch gekennzeichnet,** daß die Schaltplatteneinrichtung (9) druckempfindliche Schaltplatten (7, 8) enthält.

6. Medizinisches Gerät nach einem der Ansprüche 1 bis 5, **dadurch gekennzeichnet,** daß die Schaltplatteneinrichtung (21) kapazitiv wirkende Schaltplatten (S1,1 bis S12,6) enthält.

7. Medizinisches Gerät nach einem der Ansprüche 1 bis 5, **dadurch gekennzeichnet,** daß die Schaltplatteneinrichtung (9; 21) am Boden (3; 17) oder an einer Wand des Aufstellungsraumes des medizinischen Gerätes anbringbar ist.

FIG 1

FIG 2

FIG 3

FIG 4

FIG 5

S1,1
21

S12,6

H
V

27
I/O-PORT

20

19

24
22
25
ROM
23
RAM
26

W1
W2

T1
T2

M1
M2

EP 0 445 330 A1

12

**Europäisches Patentamt**

# EUROPÄISCHER RECHERCHENBERICHT

Nummer der Anmeldung

**EP 90 10 4373**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X,D,Y | DE-A-2 149 240 (SIEMENS A.G.) <br> * Seite 3, Zeile 11 - Seite 4, Zeile 13 * * Seite 5, Zeile 1 - Seite 7, Zeile 14 * * Ansprüche 1-4; Figur * <br> — — — | 1,5,7,2 | A 61 B 6/10 <br> F 16 P 3/12 |
| Y,A | DE-A-3 604 955 (SIEMENS A.G.) <br> * Spalte 2, Zeile 1 - Spalte 3, Zeile 67 * * Ansprüche 1-4; Figuren * <br> — — — | 2,4 | |
| A | US-A-4 303 829 (W. WAGNER) <br> * Spalte 6, Zeile 57 - Spalte 8, Zeile 5; Figuren 3, 4 * <br> — — — | 1,3-5 | |
| A | DE-A-2 529 475 (K. NICOL + E.M.C. HENNIG) <br> * Seite 5, Zeilen 7 - 16; Figur 1 * * Seite 6, Zeile 27 - Seite 7, Zeile 11 @ Seite 9, Zeile 18 - Seite 10, Zeile 17 * <br> — — — — — | 3,5-7 | |

| RECHERCHIERTE SACHGEBIETE (Int. Cl.5) |
|---|
| A 61 B <br> F 16 P <br> B 23 Q |

Der vorliegende Recherchenbericht wurde für alle Patentansprüche erstellt

| Recherchenort | Abschlußdatum der Recherche | Prüfer |
|---|---|---|
| Den Haag | 04 November 90 | RIEB K.D. |